# EUROPEAN PATENT APPLICATION

(11) **EP 4 811 370 A1**
(43) Date of publication of application: **23.09.2026**
(21) Application number: 24927623.9
(22) Date of filing: 06.08.2024
(51) Int. Cl.: G16H 20/00, G16H 40/60, G16H 50/20

(54) **SYSTEM AND METHOD FOR PROVIDING HEALTH ADVICE**

(71) Applicant: IIDA Group Holdings Co., Ltd., Musashino-shi, Tokyo 180-0013 (JP)
(72) Inventor: MORI, Kazuhiko, Musashino-shi, Tokyo 180-0013 (JP); MATSUMOTO, Koichi, Musashino-shi, Tokyo 180-0013 (JP)
(74) Representative: Mewburn Ellis LLP
(86) International application number: PCT/JP2024/028144
(87) International publication number: WO 2026/033652

(57) **Abstract**

To provide a system and method that can effectively utilize biometric data obtained in the daily life of the user for the user's maintenance and improvement of the health, and can provide further customized user-friendly health advice for the user, a system for providing health advice for a user includes: a structure installed in a facility and including a sensor that continuously collects biometric data of the user, an analyzing device so configured as to determine the health condition of the user by performing analysis of the biometric data collected by the sensor, a program generating device so configured as to generate a health promotion program in response to the user by using the results of the determination of the health condition, and an advice generating device so configured as to input the determined health condition, the generated health promotion program, and additional information relating to the user into a large language model to obtain health advice for the user.

## Description

### TECHNICAL FIELD

The present invention relates to a system and method for providing health advice in response to the health conditions of a user of a facility.

### BACKGRAUND ART

In recent years, a smart house has been attracting attention. The smart house generally refers to a house that uses information technology (IT) to control appliances that use electricity and gas, such as lighting fixtures, cooking appliances, and air conditioning equipment, thereby to optimally control energy consumption.

In Patent Document 1 (Japanese Patent No. 7285886), the present applicant proposed a system for providing health promotion program that characterized by including a sensor that is installed in a house and continuously collects biometric data of the resident of the house, an analysis device that analyzes the collected biometric data and determines the health condition of the resident, and a program generation device that generates a health promotion program appropriate to the resident based on the results of the health condition determination, and a method for providing health promotion program corresponding to the system.

The system and method have the advantage that biometric data obtained in the daily life of the user can be effectively utilized for the user's maintenance and promotion of the health.

### PRIOR ART REFERENCE

### Patent Reference

Patent Document 1: Japanese Patent No. 7285886

### SUMMARY OF THE INVENTION

### Problem to be solved by the invention

The applicant has further investigated and found a method for providing users with more customized and user-friendly health advice.

That is, an object of the present invention is to provide a system and method that can effectively utilize biometric data obtained in the daily life of the user for the user's maintenance and improvement of the health, and can provide further customized user-friendly health advice for the user.

### MEANS TO SOLVE THE PROBLEMS

In order to solve the above-mentioned problems, one embodiment of the present invention is to provide a system which is a system for providing health advice for a user, and includes:
a structure installed in a facility and including a sensor that continuously collects biometric data of the user,
an analyzing device so configured as to determine the health condition of the user by performing analysis of the biometric data collected by the sensor,
a program generating device so configured as to generate a health promotion program in response to the user by using the results of the determination of the health condition, and
an advice generating device so configured as to input the determined health condition, the generated health promotion program, and additional information relating to the user into a large language model to obtain health advice for the user.

In the system of the present invention, it is preferable that the additional information includes at least one of the following kinds of information: basic information including at least one of the user's age, sex, and nationality; biological information including at least one of the user's height, weight, body composition, basal metabolism, and blood pressure; external information including at least one of the financial and economic situation, logistics, seasons, trends, temperature, and humidity; and unique information including at least one of the user's hobbies and preferences, preferences for things and foods, allergies, physical characteristics, personal or household economic situation, and mood of the day.

Further, in the system of the present invention, it is preferable that the additional information includes the response of the user to the health advice.

Further, in the system of the present invention, it is preferable that the biometric data includes at least one kind of data which show facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components.

Further, in the system of the present invention, it is preferable that
the analysis device is so configured as to use the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person are used as a teaching data, to use a predictive model obtained by machine-leaning a leaning model where the input is the human biometric data, and the output is the health condition of the person, and to output the health condition of user from the collected biometric data.

Further, in the system of the present invention, it is preferable that
the program generation unit is so configured as to perform the procedures where:
the biological characteristics of the user are extracted by comparing the collected biometric data with a predetermined evaluation index,
the health promotion program for the user is generated from the collected biometric data by using the predetermined evaluation index and the evaluation values indicating the effectiveness of the health promotion program as a teaching data, and by using an evaluation model obtained through a machine leaning by using a leaning model where the input is the biometric data and the output is the evaluation to the health promotion program, and
the extracted biological characteristics and the generated health promotion program are output.

. Further, the other embodiment of the present invention provides a method characterized in that,
in the method for providing a health promotion program for a user, a computer performs the procedures of:
conducting an analysis of biometric data of the user collected at a sensor installed in a structure in a facility to determine a health condition of the user,
generating the health promotion program in response to the user by using the results of the determination of the health condition, and
inputting the determined health condition, the generated health promotion program, and additional information relating the user into a large-language model to obtain a health advice for the user.

### EFFECT OF THE INVENTION

In accordance with the present invention, it is possible to provide the system and method that can effectively utilize biometric data obtained in the daily life of the user for maintaining and improving the health of the user, and can provide further customized user-friendly health advice for the user.

### BRIEF EXPLANATION OF DRWAINGS

FIG. 1 is a schematic diagram showing the overall image of an embodiment of the present invention.
FIG. 2 is diagram showing an embodiment of a check-up gate including the display device 10.
FIG. 3 is another embodiment of display device 10.
FIG. 4 is other embodiment of the display device 10.
FIG. 5 is a block diagram showing the overall configuration of the system 1 which provides the health advice.
FIG. 6 is a flowchart showing the processing procedures which provide the health advice.
FIG. 7 is a diagram showing the overall image of the procedures including the generation and provision of health advice and the response by the user as to the health advice.

### MODE FOR CARRYING OUT THE INVENTION

In the following, by referring to the drawings, the typical embodiments of the system and method in accordance with the present invention are explained in detail. However, the present invention is not particularly limited to the embodiments shown in the drawings. Since these drawings are presented to explain the concept of the present invention, there are cases where ratios and numbers are exaggerated or simplified as necessary for ease of understanding.

### 1. Overall image of the present embodiment

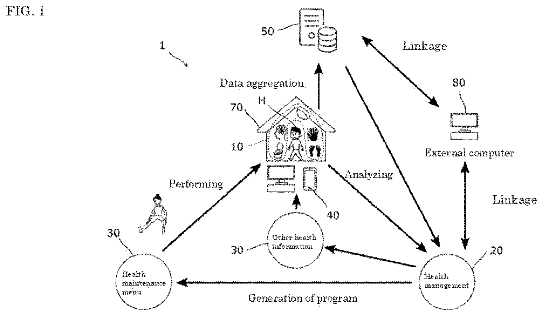

The overall image surrounding the system 1 in accordance with the present embodiment will be described with reference to FIG. 1.

The facility 70 in the present embodiment (the present invention) is a concept that includes any facility (also referred to as facility, institution, site, place, or activity base, etc.) in which the system 1 can be utilized. Examples of the facility 70 include residences, workplaces (office, factory, etc.), educational facilities such as schools, medical facilities such as hospitals and health checkup centers, nursing facilities, care homes, nursing homes, residential facilities for the elderly, exercise facilities, recreational facilities, gathering places, accommodation facilities, station building, and airfield. The facility 70 may further include mobile objects such as vehicles, ships, and aircraft.

When passing through the predetermined sections of these facilities 70 or staying in the predetermined areas, various sensors are installed at various locations in the peripheral portions of these sections or areas, and the biometric data of the user is sensed at these sensors. In the present invention, a structure in which various sensors are installed at the above-mentioned locations or peripheral portions of the area is referred to as a "check-up gate". The types of sensors available here are, for example, an image sensor, a temperature sensor, a weight sensor, and the like, the details of which will be described later. These sensors may be installed in the facility 70 on their own, or they may be incorporated into the display device 10 described below, which forms the basis of the check-up gate.

Here, the biometric data refers to all data obtained directly from the user or indirectly by applying a predetermined process, and includes, for example, image data obtained by photographing the user and data obtained by analyzing the image data.

The biometric data includes not only dynamic data such as walking posture, walking speed, change in joint range of motion, fluctuation in center of gravity and amount of activity, but also time-series data such as myoelectricity, electrocardiography and brain waves, static data such as standing and sitting posture, body temperature, blood pressure and blood flow, heart rate, breathing, sweating, eyeballs, sleeping time, amount and time of excretion, blood components, urine components such as blood sugar level, saliva components, intraoral (tongue, pharynx, etc.) images, fecal components, facial images, skin moisture and oil content.

The biometric data may be stored in a database (DB) along with other types of data. The database (DB) may be created on a computer belonging to the system 1 or on an external computer 80.

The system 1 obtains the biometric data of the user via the display device 10 and other devices 30, and evaluates the health condition the user by computer analysis of the obtained biometric data. All or part of the computer analysis described above may be performed by the display device 10, or by other computers in the facility 70 or the external computer 80. In this case, the results of the analysis may be transmitted from the other computer or external computer 80 to the display device 10.

One example of the health evaluation, which will be described in detail later, includes tongue examination, cognitive function / stress check, blood pressure, infectious disease screening, dementia prediction, posture control prediction, mineral balance check, grip strength estimation, body composition estimation, lower limb joint disease estimation, heart rate / pulse wave estimation, and customized functions are provided in accordance with the location or location where the display device 10 is introduced. Moreover, the biometric data is naturally acquired in the life of the user.

The health promotion program customized for each user is generated based on the determination result of the health condition. Here, the generated health promotion program includes, other than exercise programs, various programs and various health information for maintaining and promoting the user's physical and mental health.

To give an example, for the user who is evaluated as being under excessive strain at the workplace, efforts to alleviate the strain, such as improving the work, taking holidays, exercising moderately, getting enough sleep, and consulting a medical doctor, can be suggested. Alternatively, an exercise menu may be presented in accordance with the health condition of the user.

Alternatively, for the user who is evaluated to be in the state of insufficient exercise at the workplace, for example, the moderate exercise can be suggested.

Alternatively, for the user who is suspected of being undernourished or overnourished, a diet menu can be suggested.

Alternatively, for the user who is evaluated as having concerns about cognitive function, a consultation with a medical doctor can be suggested.

Alternatively, for the user who is suspected of suffering from an infectious disease, a consultation with a medical doctor can be suggested.

Alternatively, for the user who is evaluated as having a mineral imbalance, an improvement of diet or consultation with a medical doctor can be suggested.

Such a health promotion program is provided to the user together with the evaluation result of the health condition, and the user performs the program.

Then, by repeating the above-mentioned sensing, evaluation of health condition, creation of the health promotion program, and performance of the program by the user, the health function of the user is improved.

For example, when the user simply stands in front of the display device 10, the display device 10 acquires various health data relating the user. Since the display device 10 can be used as an ordinary mirror, it is possible to be naturally incorporated into daily life while ensuring the mirror image quality which can be used.

In the display device 10 (or external computer 80), an artificial intelligence model integrates and analyzes the health data stored in the data server and the health data sensed by the display device 10 on a daily basis. As a result, it is possible to detect physical anomalies that the user, his or her family, medical personnel, or caregivers cannot easily notice, and to provide health advice and alert for each user. Thereby, it is possible to help improvement of pre-illness and improvement of health.

At this time, it is suitable to input the determined health condition, the generated health promotion program, and additional information relating the user into the large language model to obtain customized health advice for the user. Thereby, the user-friendly health advice can be obtained, and it is expected to improve the understanding of the user of the health advice, and to practice easily the health advice by the user.

At the same time, the collected biometric data, the results of the health condition evaluation, and the created health promotion program are consolidated on a data server or the like, and with the consent of the user, may be presented to medical personnel such as a medical doctor, and utilized by the medical personnel. Further, the generated evaluation result of the health condition may be presented to and utilized by medical personnel with the consent of the user.

Furthermore, the collected biometric data and the generated determination result of the health condition may be used by the government and the local community, with the consent of the user, to create a more comfortable and livable community, to manage the health of residents, and to enable them to continue to live independently at home in old age.

### 2. Details of the system relating the present embodiment

The system 1 in accordance with the present embodiment realizes a sensing function for collecting biometric data from the user in the facility 70, an analysis and determination function for determining the health condition of the user by analyzing the collected biometric data, a program generation function for generating a health promotion program based on the determination result of the health condition, and an advice generation function for obtaining a health advice for the user by inputting the determined health condition, the generated health promotion program and the additional information relating the user into the large language model.

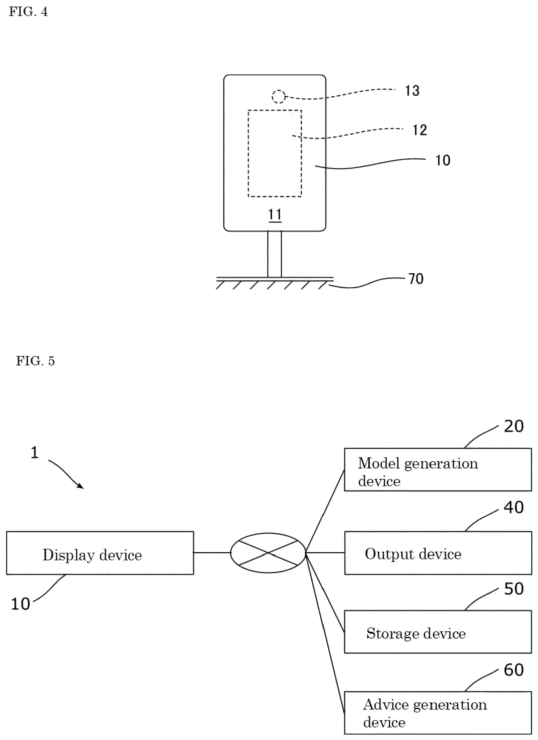

In order to realize such functions, the system 1 includes the display device 10 and a model generating device 20 (see FIG. 5). The system 1 may further include an output device 40, a storage device 50, and an advice generating device 60. These components are communicatively connected via a wired or wireless communication network. The display device 10, the model generating device 20, the model generating device 20, the output device 40, the storage device 50, and the advice generating device 60 may be separate or may be integrally configured.

The storage device 50 may be installed as a component of the data center (not shown), and the data center may store the collected data as well as the collected samples (e.g., saliva and fecal samples). The data center in this case can also be called a biobank.

In the following, these components are described in detail.

### 2-1. Display device

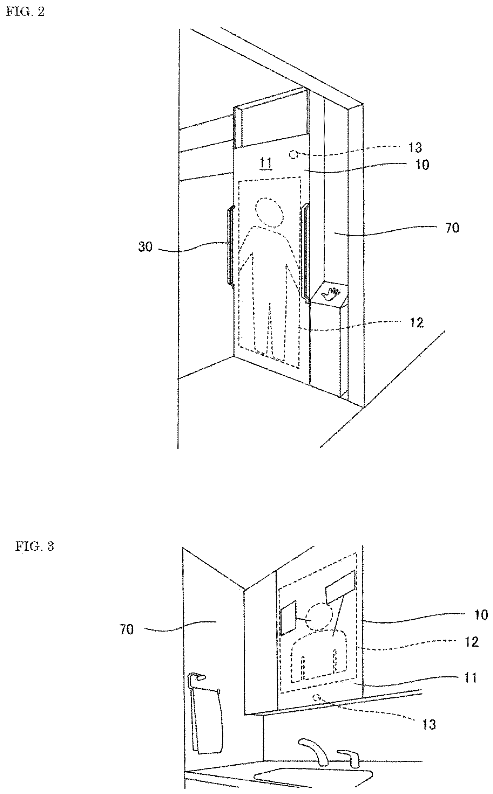

The display device 10 is described with reference to FIG. 2 to FIG. 4. FIG. 2 to FIG. 4 show various embodiments of the display device 10.

The display device 10 can display various characters and images.

The display device 10 may be installed in the facility 70 or may be placed in the facility 70. As the place for installing the display device 10, examples include an entrance, a washroom, a living room, or a living space. Further, examples include an entrance in an apartment building, an entrance or a living room in an accommodation facility, an entrance or an office room in an office, a fitness or a sports gym, a beauty salon, and a beauty counter.

The display device 10 can acquire various biometric data. The biometric data that can be acquired by the display device 10 has been described above. The display device 10 may, for example, automatically start the acquiring of the biometric data when it is determined that the user has approached a predetermined range from the display device 10 based on the detection result of the motion sensor, or may start the acquiring of the biometric data in response to the operation of the display device 10 by the user. Examples of the operation of the user include pressing or touching a button on the display device 10, but are not limited thereto.

For example, the display device 10 may be composed by including a mirror surface 11, a display 12, an image sensor 13 (image sensor), a processor, a memory, and a communication device. It can be said that this type of display device 10 is an Internet of Things (IoT) device called a smart mirror.

That is, the display device 10 has the mirror surface 11. The mirror surface 11 can reflect the user's whole body, the upper body, or mainly the face. The mirror surface 11 can be configured, for example, as a laminate of transparent plate glass and a metal reflective surface (back surface mirror).

A display 12 is incorporated into the mirror surface 11. The display 12 may be, for example, a liquid crystal display, may be an organic EL display, or may be a video projector screen.

An image sensor 13 is embedded or attached to the mirror surface 11. The image sensor 13 acquires image data by photographing the user who is in front of the display device 10. The image sensor 13 is, for example, a semiconductor image sensor, which may be a CCD image sensor or a CMOS image sensor.

The wavelength range of the image sensor 13 is not limited to visible light. That is, the image sensor 13 may be an infrared temperature camera, a hyperspectral imaging camera, or an RFID sensor.

The image data acquired by the image sensor 13 is transmitted to a processor and memory (not shown), and various processes are performed or stored. The processor may include, for example, a central processing unit (CPU), a graphics processing unit (GPU), or a microprocessor. The memory may include random access memory (RAM) and read-only memory (ROM).

Here, examples of the processing applied to the acquired biometric data and the results of the processing are as follows:
- Estimation results of oral microflora or intestinal microflora obtained from analysis of the tongue images, and evaluation of disease risk based on the estimation results
- Evaluation of cognitive function and stress predicted from facial expression / gaze / pulse wave obtained from analysis of the facial images
- Detection of body temperature, heart rate variability, and CO₂ exhaled volume obtained from analysis of the facial image, and health checks and screening for infectious diseases based on the results of the detection. Note that, the facial image can be used for personal identification and authentication.
- Detection of amyloid-ß obtained from analysis of the palm image, and evaluation of dementia risk predicted from the amount of amyloid-ß accumulation. Note that, the palm image can be used for personal identification and authentication.
- Detection of dizziness or lightheadedness obtained from analysis of the whole body image, and prediction of abnormalities in nervous system function based on the result of the detection
- Detection of toxic metals or minerals from analysis of the facial or palm image, and evaluation of the mineral balance of the body based on the detection result

The process described above can be performed, for example, as follows.

That is, from the data accumulated in the data server (storage device 50), a pair of sensing data and correct answer label (correct answer data, teacher data) for them are created, and machine learning (for example, deep learning) by using an artificial intelligence (AI) model is performed. The machine learning is repeated to extract the features of the data and predict the health condition of the user.

As described above, the featured information extracted by the AI model (i.e., the determined health condition and the generated health promotion programs) and the additional information may be input into the large language model (LLM) and converted into more user-friendly information. The display device 10 may perform the generation of health advice by the large language model itself, or may work with the external computer 80 capable of performing the large language model, for example, via an application programming interface (API). Details of the large language model and the generation of health advice by the large language model will be described later.

Return to the description of the display device 10. The display device 10 can present the converted information to the user. For example, there includes the displaying on the display 12 or the reding aloud the text.

Further, by having the above-mentioned AI model learn the reaction of the user at that time, it is possible to present more accurate and personalized information, which leads to a change in the behavior of the user.

The above-mentioned analysis may be realized by performing various analysis programs and various biometric data stored in the memory on the processor. Alternatively, the analysis result may be obtained by applying various biometric data to the learned model stored in the memory. Further, an appropriate preprocessing may be applied before the analysis is performed.

Alternatively, the analysis may be performed by the external computer 80, and the analysis result may be returned to the display device 10.

The acquired image data, preprocessed data, or analysis result may be stored in the memory, or may be transmitted via a communication device to another mirror device, the external computer 80, or the storage device 50. The communication device may be a wired communication device or a wireless communication device. The wired communication device includes, for example, an Ethernet adapter and a modem, and the wireless communication device includes, for example, Bluetooth (trademark), Wi-Fi, Thread, ZigBee, Matter, and low-power wide-area wireless (LPWA). The LPWA includes, for example, LTE Category M1, NB-IoT, LoRaWAN, Sigfox, Wi-SUN, ZETA, and ELTRES, and the like.

In addition, the display devices 10 may also include other input devices such as a touch panel (including one with a non-contact interface) and a microphone, and other output devices such as a speaker.

Therefore, the display device 10 can be utilized not only as a mirror, but also as a collection device for the biometric data, a display device for collected and analyzed biometric data, various information and contents, and furthermore as a communication tool.

For example, the display 12 embedded in the mirror surface 11 can project an image onto the surface of the mirror, and this image includes that photographed by the image sensor 13. Further, the display 12 can also display the acquired biometric data. Examples of the display method include graphic displays such as graphs and illustrations other than the character displays.

The display device 10 can perform the facial recognition and personal authentication by the built-in image sensor 13 or another sensor. Further, the display device 10 can perform the voice recognition and environmental measurement, when having a built-in microphone, temperature / humidity sensors.

The display device 10 can communicate with other devices 30 in the facility 70 via the built-in communication device. The other devices 30 include other display devices 10, PCs, and portable terminals disposed in the same facility 70. The portable terminals include, for example, smartphones, tablets, and wearable devices. Further, the display device 10 can communicate with the external computer 80 via the Internet. The external computer 80 includes servers, PCs, and portable terminals located outside of the facility 70.

When the display device 10 has a built-in speaker, it is possible to provide audio output. Further, when the display device 10 is equipped with an input device such as a touch panel, it is possible to be easily operated, for example, by touching the surface of the mirror.

In this way, the display device 10 evaluates the health condition of the user by acquiring various biometric data and performing computer analysis of the acquired biometric data. However, all or part of the computer analysis may be performed by another computer (for example, the external computer 80) and the analysis result may be returned to display device 10.

The display device 10 can acquire the biometric data of the user at different times (first and second times) to obtain a time-series biometric data. Then, by analyzing the time-series biometric data, a time-series evaluation of the health condition can be performed.

Alternatively, by comparing the same type of biometric data (first and second biometric data) sensed at different facilities 70 (first and second facilities 70; for example, home and workplace), the health condition of the user in different environments can also be accurately evaluated.

To give a specific example of the latter, when the measured values of the user in blood pressure, heart rate, respiratory rate, and perspiration at the workplace of the user are greater than the corresponding values at the home of the user by more than a set value, the user can be evaluated as being under excessive strain at the workplace.

Alternatively, when the measured values of the user in blood pressure, heart rate, respiratory rate, blood flow, and perspiration at the exercise facility are greater than the corresponding values at the home of the user by a predetermined acceptable range, the user can be evaluated as performing moderate exercise.

Alternatively, if the measured values or estimated values of the user in moved distance, speed of movement, and metabolism at the workplace are equal to or less than the corresponding values at the home of the user, the user can be evaluated as being in a state of lack of exercise at the workplace.

Alternatively, when the measured values of the user in blood pressure, heart rate, and respiratory rate at the accommodation and recreational facility are equal to or less than the corresponding values at the home of the user by more than a set value, the user can be evaluated as being in a moderately relaxed state at the accommodation and recreational facility.

Alternatively, when the measured value of the user in the electroencephalogram in the educational facility or workplace of the user is different from the corresponding value in the home of the user, the user can be evaluated as being in a state of lack of concentration.

### 2-2. Other devices

Other devices 30 may be installed in the facility 70. The display device 10 may be combined or linked with the other devices 30.

The other devices 30 include, for example, a body composition meter, a grip strength sensor, a pressure sensor, a motion capture device, a temperature sensor, a humidity sensor, a component analyzer, a weight sensor, a flow sensor, a position sensor, an infrared temperature camera, a hyperspectral imaging camera, an RFID sensor, a motion capture device (none of which are shown), but are not limited thereto. Note that, the display device 10 may include these devices. In the example of FIG. 2, the display device 10 has a grip force sensor that takes the form of a handle as an example of one of the above-mentioned other devices 30.

The other devices 30 also continuously collect the biometric data of the user H and transmit and store the collected biometric data, together with the date and time of collection, in the storage device 50. The sensing of the biometric data of the user H by the other device 30 may be performed, for example, by constant measurement.

For example, the body composition meter is installed at the foot of the display device 10, and can measure the user's weight, BMI, body fat percentage, visceral fat level, muscle mass, body water percentage, basal metabolic rate, estimated bone mass, and the like. The body composition meter may be embedded into the floor of the facility 70 so as to be flush with the floor surface. The body composition meter may also be incorporated into a hand rail. The body composition meter acquires the body composition data of the user, for example, the foot measurement pressure on floor, and transmits the acquired data to the display device 10. The display device 10 predicts lower limb joint disease based on changes in the foot measurement pressure on floor.

The grip strength sensor is, for example, rod-shaped and is installed beside the display device 10. The grip strength sensor may also serve as a gripping member or a handrail. The grip strength sensor detects the pressure applied to the grip strength sensor when the user grips the grip strength sensor, and transmits the pressure data to the display device 10. The display device 10 calculates the grip strength by using this pressure data and a skeletal analysis based on the whole body image of the user. Then the display device 10 predicts the frailty of the user by using the change in grip strength and the walking speed calculated from the whole body image of the user.

The infrared temperature camera is, for example, installed in living spaces, such as a living room, study, bedroom, and working room of the facility 70, and can measure the user H's facial expression, heart rate, oxygen saturation, carbon dioxide exhalation, surface body temperature, and deep body temperature.

The hyperspectral imaging camera is, for example, installed at or near the entrance, and in addition to the personal authentication function of user H, can perform the user H's skin protein analysis, the measurement of body composition (including, for example, the composition ratio of fat / bone / lean soft tissue, body weight, body fat, basal metabolism, visceral fat, muscle mass, and bone mass), the measurement of autonomic nerve (including, for example, autonomic fatigue and balance between sympathetic nerve and parasympathetic nerve). Alternatively, the hyperspectral imaging camera is installed at the dining table, in the kitchen, and near them, and can measure the glycohemoglobin (HbA1c).

The RFID sensor is installed on the wall of the room, and can measure the amount of water in the body of user H.

The pressure sensor is, for example, installed on the floor of the corridor and room, and can measure the foot pressure distribution of the user H.

The motion capture devices is, for example, installed in the corridor of the room, and can measure the user H's walking posture, walking speed, joint range of motion change, variation of gravity center, and activity amount.

The other device 30 may be installed hidden from public view so that the user H cannot immediately discover it. This is to prevent the user H from becoming nervous when he or she notices the presence of the other device 30 and to suppress the sensed biometric data from deviating from the daily values of the user H. In other words, to know the user H in his or her natural state in daily life.

Further, various sensors may be installed to obtain environmental data. That is, a thermometer for measuring room temperature, a hygrometer for measuring room humidity, an illuminance meter for measuring room brightness, a CO₂ meter for measuring room CO₂ concentration, a noise sensor for measuring environmental sound, and a light sensor for detecting light conditions in the environment may be installed. The environmental data collected at these sensors is transmitted to and stored in the storage device 50. The display device 10 may include these sensors.

Additionally, the biometric data of the user may be acquired by using various sensors built into a wearable device and a portable information terminal such as a smartphone. This type of biometric data is useful as an aid in determining the health condition of the user.

The collected biometric data, environmental data, and other data are transmitted to and stored in the storage device 50. The storage device 50 may store the determination results of the health condition described later and the generated health promotion program. The various data stored in the storage device 50 may be made available on the external computer 80 used by the medical doctor and medical organization in charge of the user H with the permission of the user H.

Through such medical coordination, various health records necessary for diagnosis and treatment of the user H can be shared among the medical personnel in a timely manner. Of course, the data linkage may also target medical doctors, dentists, pharmacists, dieticians, or caregivers, and is not limited to these medical professionals.

### 2-3. Model generation device

The model generation device 20 is a computer that generates various statistical models. The model generation device 20 can also perform various processes by using the generated statistical models.

As an example of the statistical models, a statistical model for analyzing the biometric data and a statistical model for generating the health promotion programs are described below.

### 2-3-1. Analysis model of biometric data

The model generation device 20 can generate a predictive model for analyzing biometric data collected by the display device 10 and other devices 30. The model generation device 20 may utilize the AI analysis for the data analysis.

Specifically, the model generation device 20 uses the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person as a teaching data, and generate a predictive model where the input is the human biometric data, and the output is the health condition of the person by using a machine learning. And the model generation device 20 outputs the health condition of the user from the collected biometric data by using the predictive model. Examples of available machine learning include deep learning, but is not limited thereto.

For example, the model generation device 20 can use at least one kind of data among the biometric data such as human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, changes in joint range of motion, fluctuations in center of gravity, and amount of activity, as a teaching variable (teaching data) as an existing index, and then can conduct a machine learning by using the biometric data of the same type as the collected data as explanatory variables to generate an analysis program.

Further, the model generation device 20 can also algorithmically analyze the biometric information such as facial expressions, heart rate, oxygen saturation, carbon dioxide exhaled amount, surface body temperature, core body temperature, skin protein analysis, body composition, autonomic nerves, HbA1c, internal water content, foot pressure distribution, and the motor function information such as joint angle during walking and the time-related change thereof, lower limb muscle strength, center of gravity, or three dimensional moving distance of the observation point correlated thereto, walking posture, walking speed, and amount of activity, by using the basic information such as age, gender, height and weight, as parameters to be utilized. That is, the model generation device 20 obtains the objective evaluation including the health function and motor function as the health condition of the user H, by applying the sensing data to the above-mentioned model.

The model generation device 20 can transmit the generated analysis model to the display device 10 or the storage device 50. Further, the model generation device 20 can transmit the determination result of the health condition of the user H to the display device 10, the output device 40, or the storage device 50. The model generation device 20 can further transmit the result of the determination of the health condition of the user H to the terminals of the user and his or her family members. Then, by outputting the determination result of the health condition of the user H to the display device 10 or the output device 40, the user H and his or her family members can detect the illness or poor physical condition of the user H at an early stage and can make use for the health management of the user H.

Needless to say, such determination results can be effectively used by related parties, including medical personnel and caregivers, with the approval of the user H.

Thereby, the determination result can be effectively utilized for the support of the user H and for cooperation with related parties.

### 2-3-2. Generation Model of Health Promotion Programs

The model generation device 20 can also generate a customized health promotion program for each user H by using the determination result of the health condition. An example of a statistical model for generating the health promotion program is described below.

That is, the model generation device 20 extracts the biological characteristics of the user by comparing the collected biometric data with the predetermined evaluation index. Then, the model generation device 20 uses the predetermined evaluation index and the evaluation values indicating the effectiveness of the health promotion program are used as the teaching data, and generates the evaluation model where the input is the biometric data, and the output is the evaluation to the health promotion program by using the machine learning. The generated evaluation model may be transmitted to the storage device 50 or the display device 10.

The model generation device 20 may also use the evaluation model to output the health promotion program to be presented to the user from the collected biometric data, together with the biometric features of the user.

More specifically described, the health promotion programs are roughly classified into those related to health functions and those related to exercise functions.

For example, when generating the program relating to the health functions, the model generation device 20 clarifies the biological features of the user H by comparing the normal range for each individual with evaluation indicators such as epidemiology and medical guidelines, with respect to the facial expressions, heart rate, oxygen saturation, carbon dioxide exhaled amount, surface body temperature, core body temperature, skin protein analysis, body composition, autonomic nerves, HbA1c, internal water content, and foot pressure distribution. Then, the model generation device 20 can construct the algorithm (evaluation model) related to each individual lifestyle habits by performing the AI analysis on the measured data, the basic information such as age, gender, height and weight, and the living environment data of the user H. Here, an example of the AI analysis is deep learning, but it is not limited thereto.

The model generation device 20 can also present the behavior change and the accompanying effects thereof by applying the constructed evaluation model to the biometric data of the user, and the like.

When generating a program relating the exercise functions, the model generation device 20 clarifies the features of the dynamic analysis of the user H by comparing the positions and velocities of each joint, joint angles and angular velocities. Then the model generation device 20 can construct the motion measurement algorithms (evaluation model), based on the measured data, in addition to estimated energy consumption and exercise effects during exercise. Here, an example of the AI analysis is deep learning, but it is not limited thereto.

The model generation device 20 can also present recommended exercise and energy consumption during exercise and exercise effects by applying the constructed evaluation model to the biometric data of the user, and the like.

The model generation device 20 can transmit the constructed evaluation model to the display device 10 or the storage device 50. Further, the model generation device 20 can also transmit the various generated programs to the display device 10, the output device 40, or the storage device 50. The model generation device 20 can further transmit the various generated programs to the terminals of the user and his or her family members.

Furthermore, the model generation device 20 may generate the own large language model. The large language model is trained to accept the inputs of the evaluation of the health condition, health promotion programs, and additional information (the above-mentioned basic information, biometric information, external information, and unique information) about a certain person, and to output the health advice for that person.

By outputting the above-mentioned program or health advice to the display device 10 or the output device 40, the user H and his or her family members can clearly grasp what they should do to maintain, recover and improve the health functions and exercise functions of the user H, that is, behavior change, and further can utilize for the health management of the user H. The program or health advice can be utilized in the health management of the user H.

Needless to say, such programs or health advice can be shared among the related parties, including medical personnel and caregivers, with the approval of the user H.

Here is an example of the behavior change.

For example, in the kitchen, the user operates a kitchen monitor or his or her own mobile terminal to activate the predetermined application program. The application program evaluates the health condition of the user by referring to the biometric data of the user, and the like, and presents menus, ingredients, cooking methods, nutrients, calorie content, and the like, which are tailored to the individual user. The application program can also acquire the image of the dish prepared by the user via the image sensor 13, to calculate and display the amount of nutrients and energy contained in the dish.

In the living space, the user operates the TV or monitor to activate an application program. The application program suggests games by using eye tracking to improve or grasp cognitive functions. When the user selects one of several suggested games, the game starts and cognitive functions are measured. During or after the game, the application program refers to the necessary biometric data and displays feedback regarding the game score and improvement of cognitive function.

In the living room, the user operates the mirror-type monitor (for example, the display device 10) to activate the application program. The application program evaluates the health condition of the user by referring to the biometric data of the user, and the like, and presents a menu of appropriate exercise load for the individual user.

A virtual trainer is displayed on the mirrored monitor, and the user follows the movements of the virtual trainer to practice the exercise menu. The application program calculates and displays the amount of energy consumption after exercise and presents feedback on exercise function.

When going out, the user operates his or her own mobile terminal to activate the application program. The application program can display the health advice along with the result of the measurement of the health data of the user.

Note that, the model generation device 20 can be configured as one or more computers including an arithmetic circuit (processor) such as central processing unit (CPU), as well as a random-access memory (RAM) and a read-only memory (ROM). The above-mentioned functions of the model generation device 20 can be realized by reading an execution program stored in the ROM into the RAM and performing by the CPU. The model generation device 20 can perform the determination of the health condition, and the generation of the health promotion program, and the generation of the health advice periodically or at a timing deemed necessary for each individual, by using continuously collected biometric data.

Alternatively, the above-mentioned functions of the model generation device 20 may be incorporated into the display device 10.

### 2-4. Advice generation device

The advice generation device 60 is a computer configured to input the determined health condition, the generated health promotion program, and the additional information relating the user into the large language model and to output the health advice for the user H. That is, the advice generation device 60 stores the learned large language model and can output the health advice in response to the input. Alternatively, the advice generation device 60 may access the external computer 80 via an API, for example, and use the learned large language model.

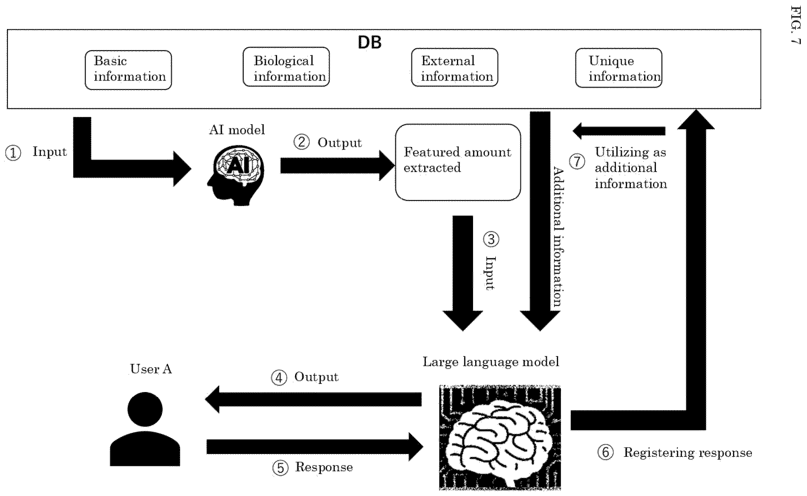

In the following, referring to Fig. 7, the generation of health advice by using the large language model is described.

The large language model is a language model built by using a large data set and deep learning technology (for example, Transformer), where the language model is modeled by using the probability of occurrence of text and words. Since the large language models can understand and generate the natural language and other contents and perform a wide range of tasks, the inventors believed that by inputting the above information into the large language model, a customized, user-friendly health advice for the user H can be obtained.

Here, as the large language models, for example, BERT (Bidirectional Encoder Representations from Transformers), GPT (Generative Pre-trained Transformer), Bard, LLaMA, Gemini, and the like can be used. In order to utilize these known models, the system 1, for example, the display device 10 may use an API ,or may use a computer within system 1.

Alternatively, the system 1, for example, the model generation device 20 may generate the unique large language model specified to the generation of health advice by machine learning.

The items to be entered into the large language model are broadly classified into the following five categories:
- Basic information: user's age, gender, nationality, etc.
- Biometric information: user's height, weight, body composition, basal metabolism, blood pressure, etc.
- External information: financial / economic information, logistics, seasons, trends, temperature, humidity, etc.
- Specific information: user's hobbies and preferences, things and foods preferences, allergies, partial body characteristics (for example, large feet for height), personal or household financial information, mood of the day, etc.
- Information extracted by the AI model

Here, the information that has already been input to the AI model may be input to the large language model.

The basic information, biometric information, external information, and unique information shall be input into the large language model as the additional information. These types of information are useful for customizing the health advice for the user H.

The additional information may include the reaction of the user to the provided health advice.

For example, considering the case that, for the user A, the large language model may output the following health advice: "As a result of measuring the nutritional status of Ms. A, we found that the user is deficient in vitamin C, so we recommend that the user takes lemon." The user A reacts to this health advice by saying that the user cannot eat lemon because of an allergy or dislike. This reaction may be obtained, for example, by input by the user A via the touch panel or microphone of the display device 10, or by motion analysis of the actions of the user A acquired by the image sensor. That is, as a result of the reaction of the user A, the food preference or allergy information for the user A, i.e., "Ms. A cannot eat lemon.," is obtained. Such information may be registered in a database.

When receiving the above-mentioned food preference or allergy information of the user A as the additional input, the large language model performs the process again and outputs the updated health advice. The updated health advice may be, for example, "Then try consuming mandarin oranges and kiwifruit. If you eat them with yogurt in the morning, they will also improve your intestinal environment. The effects of eating them for one week are as follows. ~~~". The update of the health advice may be performed at any time in response to the reaction of the user A, and there is no limit to the number of updates.

### 2-5. Output Device

The output device 40 is a device that outputs the health advice which includes the determination result of the health condition and the health promotion program. The output device 40 includes the terminal (including smartphone, personal computer), display, printer, projector, speaker of the user H. The display device 10 may also serve as the output device 40.

For example, when the determination result of the health condition indicates an abnormality in the health condition of the user H, that is, a deviation from the normal range (for example, with respect to the data expressed in numerical values, when the result is above or below the predetermined threshold, or when being out of range), the output device 40 may output an alert to the terminal of the user H and the related parties. Note that the output device 40 may display other information related to daily life, such as information regarding energy and housing, and regional information.

### 3. Operation of System

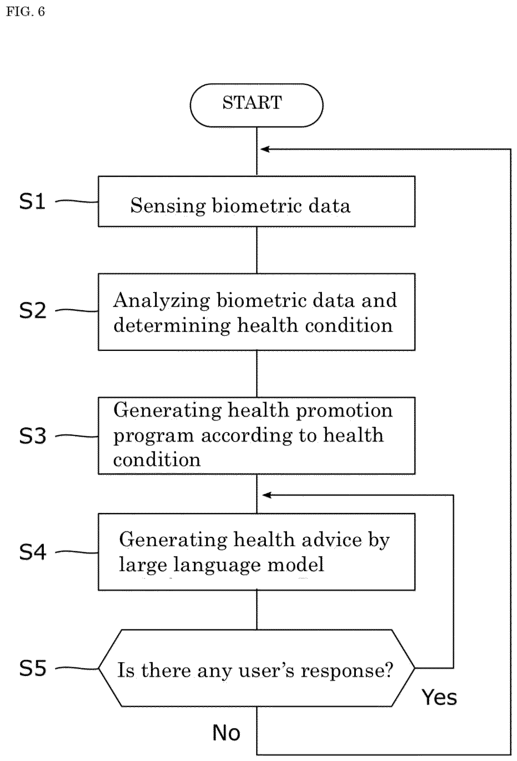

The processing procedures for providing the health promotion program by the system 1 will be explained with reference to FIG. 6.

Firstly, in step S1, the model generation device 10 and the other device 30 sense the biometric data of the user H and stores in the storage device 50 in association with the sensing time. Sensing is performed continuously.

Then, in step S2, the model generation device 20 or the display device 10 analyzes the collected biometric data, determines the health condition of the user H, and stores the determination result in the storage device 50. For example, the model generation device 20 or the display device 10 stores the leaned model, and acquires the health condition of the user H by applying the biometric data of the user H to the learned model. Step S2 is performed periodically or at a timing deemed necessary for each individual.

When the determination result of the health condition is generated, in step S3, the model generation device 20 or the display device 10 generates the health promotion program for each user H based on the determination result of the health condition, and stores the program in the storage device 50.

The determination result of the health condition and the health promotion program are displayed on the output device 40 or the display device 10. Thereby, the user H can understand own health condition (for example, health functions and motor functions) and can clearly understand what to do to maintain, recover, and improve own health functions and motor functions. The user H can practice the presented health promotion program and utilize for own health management.

Alternatively, the determination result of the health condition and the health promotion program may be input into the large language model together with other information relating the user to generate the customized health advice for the user (step S4). The health advice customized for the user is user-friendly and can be easily performed by the user.

Next, in step S5, it is determined whether or not the user has reacted to the presented health advice. If the reaction of the user is confirmed, returning to step S4, various information including the reaction of the user is entered into the large language model, and the updated the health advice is presented to the user. Since the reaction of the user may include a feature of the user which is unknown to the system 1, the updated health advice by using such reaction will be more closely related to the user.

Additionally, each time the reaction of the user is obtained, the additional information may be entered into the large language model and further updated the health advice may be presented to the user. Thereby, it is expected to further improve the accuracy of the health advice.

Alternatively, if the reaction of the user is not confirmed within the predetermined period, the process starts again from step S1.

An example of the operation is given below.

Here, the user A receives various health advice.

When the user A stands in front of the display device 10, the display device 10 identifies the individual by the face recognition or the like, and activates the predetermined application program. This application program gives various instructions to the user A by displaying a virtual person on the display 12 of the display device 10. In other words, the display device 10 senses the biometric data while proceeding with a dialogue between a virtual person (for example, a virtual butler) and the user.

When the measurement is completed, the display device 10 displays the measurement result and health advice in accordance with the selection of the user A. At this time, the display device 10 may display a virtual person (for example, a virtual medical doctor) on the display 12 and let the person explain.

Furthermore, when the user A wishes to display the exercise menu, the display device 10 may display a virtual person (for example, a virtual trainer) on the display 12 and present an exercise menu for each individual.

Here, an example of the result of the measurement and the health advice is given.

"I've been seeing a loss of muscle mass in the past few days, and I'm going to present a diet and exercise menu that suits Ms. A, so let's do our best together! By achieving, you can expect the following health benefits. • • • "

Then, return to sensing and repeat the above procedures. Thereby, the user A can effectively utilize the biometric data obtained in daily life to promote the health functions of the user A. Furthermore, the user A can understand the effects of performance of the health promotion program through the determination result of subsequent health condition.

There is give another example.

Here, considering a situation where the user B receives various health advice.

The user B sticks out his tongue in front of the display device 10 to photograph an image of his tongue. The image of the tongue of the user B is input to the tongue examination AI model along with other information such as user B's age, gender, height, and weight.

Here, the AI model of the tongue examination is a leaned model obtained by machine learning by using the image of the tongue as input and the evaluation of the health condition as the output. This model has also been learned so as to generate a health promotion program from the evaluation of the health condition and the above-mentioned other information. The evaluation of the health condition includes, for example, the prediction of oral microbiota and the gut microbiota, and the evaluation of the risk of gastrointestinal diseases. The health promotion program includes, for example, suggestion for exercise, diet, and sleep to improve the gut microbiome.

The information extracted by the AI model is input to the large language model together with the other information relating the user B (for example, basic information, biological information, external information, unique information, etc.). The large language model converts the input information into more user-friendly information and presents to user B.

For example, in addition to the basic information of the user B, when inputting the sample data of intestinal microbiota, and the health promotion programs such as exercise for improving intestinal microbiota, diet, and sleep into the large language model (for example, GPT), and the large language model responds as the following as an example of daily activity.
Breakfast examples:
   - Oatmeal (for example, whole grain oats) served with Greek yogurt and banana
   - Green tea or black coffee
Examples of activities in the morning
   - Commuting on foot or by bicycle
   - Stretching or short walks between work
Lunch examples:
   - Salad (for example, including leaf lettuce, tomatoes, cucumbers, avocados, and carrots). With olive oil and vinegar dressing
   - Whole grain bread with fumes
   - Water or herbal tea
Examples of activities in the afternoon:
   - Work (incorporate standing work)
   - Walking for about 15 minutes after lunch
Snack examples:
   - Nuts (for example, almonds and walnuts) and fruits (for example, apples and berries)
Dinner examples:
   - Grilled salmon
   - Stir-fried vegetables (for example, including broccoli, carrots, paprika and zucchini)
   - Kimchi
   - Water or herbal tea
Examples of exercise:
   - Walking or jogging for 30 minutes after work
   - Strength training twice a week (20 to 30 minutes; for example, push-ups, squats, dumbbell exercises)
Examples of activities before bedtime:
   - Warm herbal tea (chamomile, or the like)
   - Reading, meditating, listening to relaxing music
   - Refrain from using electronic devices
Sleep:
   - Maintaining a consistent bedtime, and ensuring 7 to 8 hours of sleep

Supposing that the user B has the following reaction after receiving the above health advice.
- I can't eat bananas because I'm allergic to bananas
- I want to eat noodles for lunch
- My job is a desk job, and I'm not good at muscle training
- I want to drink a beer at dinner
- Before going to bed, I want to think about games and hobby fishing instead of reading

The reaction may be performed, for example, by inserting a check mark in the response column corresponding to each activity example on the display device 10.

The reactions are registered in the DB as information characterizing the user B.

When the information related to the above reaction is added to the input information, the large language model updates the health advice as follows, for example.
Breakfast example:
   - Oatmeal. Serve with fruit (strawberries or blueberries)
   - Green tea or iced coffee
Examples of activities in the morning:
   - Commuting on foot (for example, fast walking, using stairs)
   - Stretching between work, and simple exercises to improve posture (can be done around the desk)
Lunch examples:
   - Chilled Chinese (for example, including chilled noodles, cucumbers, tomatoes, ham, eggs, Kaiware radishes, and sesame sauce)
   - Barley tea or water
Examples of activities in the afternoon:
   - Light stretching or walking for about 15 minutes after lunch
   - Regularly take a break from your desk to rest your eyes to prevent eye strain
Snack examples:
   - Mix of nuts and dried fruits (for example, including almonds, raisins and cranberries)
Dinner examples:
   - Grilled chicken or fish (for example, salmon)
   - Stir-fried vegetables (for example, including broccoli, carrots, paprika, and zucchini)
   - Kimchi as a side dish
   - One can of beer. However, choose low-alcohol or non-alcoholic drinks.
Examples of relaxation after dinner:
   - Preparing for games and fishing
   - Light stretching and deep breathing
Sleep:
   - Maintaining a consistent bedtime, and ensuring 7 to 8 hours of sleep

The updated health advice reflects the reaction of the user B to the initial health advice, and is more in line with the user B's preferences and lifestyle.

Alternatively, when there is a further reaction from the user B, input to the large language model and update the health advice may be repeated to further improve the accuracy of the health advice.

The thus accumulated sensing data, the determination result of the health condition, and the health promotion program are stored in the storage device 50 and shared among medical personnel involved with the user H with the approval of the user H. Thereby, it is expected that this will allow the medical doctors to perform diagnosis and treatment more appropriately and quickly. Such effects can be expected not only during face-to-face medical treatment but also in remote medical treatment, and more fulfilling medical linkage will be realized.

Further, by sharing the health information of the user H mentioned above among those involved in care, it is expected that more appropriate and prompt care support will be provided.

Of course, it is also possible to use the sensing data and the determination result of the health condition as a monitoring system for the user H or an abnormality detection system.

Further, by utilizing the collected biometric data and the determination result of the health condition by the government and the local community with the consent of the user H, it is possible to reflected in policies to create a more comfortable and livable community.

Since the display device 10 and the other device 30 are installed in the facility 70, there is no need to worry about attachment and detachment unlike wearable sensors. Furthermore, since the sensors are hidden from the eyes of the user H, the user H is not aware of the existence of the sensor 10. Therefore, the daily biometric data of the user H can be obtained, and the health condition of the user can be understood with high reliability.

In the above, although the typical embodiments of the present invention have been described, the present invention is not limited to these, and various design changes are possible, and all of those are included in the present invention.

### INDUSTRIAL APPLICABILITY

In accordance with the present invention, it is possible to effectively utilize the biometric data obtained in the daily life of users for maintaining and promoting the health of the users. The utilization of the biometric data includes support for the users, and information linkage with the medical and care staffs.

### Explanation of Symbols

1 System
10 Display device
20 Model generation device
40 Output device
50 Storage device
60 Advice generation device
H User

## Claims

1. A system for providing health advice for a user, including:
a structure installed in a facility and including a sensor that continuously collects biometric data of the user,
an analyzing device so configured as to determine the health condition of the user by performing analysis of the biometric data collected by the sensor,
a program generating device so configured as to generate a health promotion program in response to the user by using the results of the determination of the health condition, and
an advice generating device so configured as to input the determined health condition, the generated health promotion program, and additional information relating to the user into a large language model to obtain health advice for the user.

2. The system in accordance with claim 1, wherein
the additional information includes at least one of the following kinds of information: basic information including at least one of the user's age, sex, and nationality; biological information including at least one of the user's height, weight, body composition, basal metabolism, and blood pressure; external information including at least one of the financial and economic situation, logistics, seasons, trends, temperature, and humidity; and unique information including at least one of the user's hobbies and preferences, preferences for things and foods, allergies, physical characteristics, personal or household economic situation, and mood of the day.

3. The system in accordance with claim 1, wherein
the additional information includes the response of the user to the health advice.

4. The system in accordance with claim 1, wherein
the biometric data includes at least one kind of data which show facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components.

5. The system in accordance with claim 1, wherein
the analysis device is so configured as to use the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person are used as a teaching data, to use a predictive model obtained by machine-leaning a leaning model where the input is the human biometric data, and the output is the health condition of the person, and to output the health condition of user from the collected biometric data.

6. The system in accordance with claim 1, wherein
the program generation unit is so configured as to perform the procedures where:
the biological characteristics of the user are extracted by comparing the collected biometric data with a predetermined evaluation index,
the health promotion program for the user is generated from the collected biometric data by using the predetermined evaluation index and the evaluation values indicating the effectiveness of the health promotion program as a teaching data, and by using an evaluation model obtained through a machine leaning by using a leaning model where the input is the biometric data and the output is the evaluation to the health promotion program, and
the extracted biological characteristics and the generated health promotion program are output.

7. A method for providing a health promotion program for a user, wherein a computer performs the procedures of:
conducting an analysis of biometric data of the user collected at a sensor installed in a structure in a facility to determine a health condition of the user,
generating the health promotion program in response to the user by using the results of the determination of the health condition, and
inputting the determined health condition, the generated health promotion program, and additional information relating the user into a large-language model to obtain a health advice for the user.
